(19) Europäisches Patentamt / European Patent Office / Office européen des brevets

(11) EP 2 739 137 B1

(12) **EUROPÄISCHE PATENTSCHRIFT**

(45) Veröffentlichungstag und Bekanntmachung des Hinweises auf die Patenterteilung:
**07.06.2017 Patentblatt 2017/23**

(21) Anmeldenummer: **12745404.9**

(22) Anmeldetag: **31.07.2012**

(51) Int Cl.:
***A01N 43/90*** *(2006.01)* ***A61K 8/04*** *(2006.01)*
***A61K 8/49*** *(2006.01)* ***A61Q 5/02*** *(2006.01)*
***A61Q 19/10*** *(2006.01)* ***C09D 7/00*** *(2006.01)*
***C11D 3/22*** *(2006.01)* ***C11D 17/00*** *(2006.01)*

(86) Internationale Anmeldenummer:
**PCT/EP2012/003244**

(87) Internationale Veröffentlichungsnummer:
**WO 2013/017255 (07.02.2013 Gazette 2013/06)**

(54) **VERWENDUNG VON ISOSORBIDMONOESTERN ALS VERDICKER**
USE OF ISOSORBIDE MONOESTERS AS THICKENERS
UTILISATION DES MONOESTERS D'ISOSORBIDE COMME AGENT EPAISSISSANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.08.2011 DE 102011109437**

(43) Veröffentlichungstag der Anmeldung:
**11.06.2014 Patentblatt 2014/24**

(73) Patentinhaber: **Clariant International Ltd**
**4132 Muttenz (CH)**

(72) Erfinder:
- **PILZ, Maurice, Frederic**
  **60329 Frankfurt am Main (DE)**
- **KLUG, Peter**
  **63762 Großostheim (DE)**
- **SCHERL, Franz-Xaver**
  **84508 Burgkirchen (DE)**

(74) Vertreter: **Paczkowski, Marcus et al**
**Clariant Produkte (Deutschland) GmbH**
**Patent & License Management Chemicals**
**Industriepark Höchst / G 860**
**65926 Frankfurt am Main (DE)**

(56) Entgegenhaltungen:
**EP-A1- 2 239 315** **WO-A2-2010/108738**
**WO-A2-2010/136121**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 739 137 B1

**Beschreibung**

Die vorliegende Erfindung betrifft die Verwendung von Isosorbidmonoestern als Verdicker.

In der Industrie werden Verdicker verwendet, um die Viskosität von Produkten wie beispielsweise von kosmetischen, dermatologischen oder pharmazeutischen Zusammensetzungen, Pflanzenschutzformulierungen, Wasch- oder Reinigungsmitteln oder Farb- oder Anstrichmitteln gezielt einzustellen. Es sind zahlreiche Verdicker für diesen Zweck bekannt. Diese Verdicker können synthetisch hergestellt worden sein oder auf natürlichen Rohstoffen basieren.

Nachteilig an der Verwendung vieler synthetisch hergestellter Verdicker, z. B. synthetischer Polymere, ist jedoch, dass ihre Herstellung oftmals aufwändig ist und auf synthetischen Rohstoffen basiert. Nachteilig an der Verwendung vieler auf natürlichen Rohstoffen basierenden Verdickern ist demgegenüber, dass das Erscheinungsbild vieler Produkte, die derartige Verdicker enthalten, verbesserungsbedürftig ist.

Es bestand deshalb die Aufgabe, Verdicker zur Verfügung zu stellen, die die oben genannten Nachteile nicht aufweisen oder diese zumindest teilweise verbessern und zudem eine vorteilhafte Verdickungsleistung zeigen.

Überraschend wurde nun gefunden, dass diese Aufgabe gelöst wird durch Verbindungen der Formel (I)

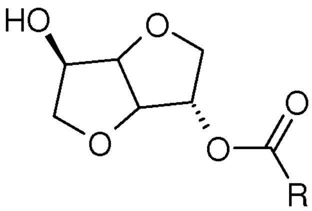

(I)

worin

R eine lineare oder verzweigte, gesättigte Alkylgruppe mit 5 bis 11, vorzugsweise 7 bis 9 und besonders bevorzugt 7 Kohlenstoffatomen oder eine lineare oder verzweigte, ein- oder mehrfach ungesättigte Alkenylgruppe mit 5 bis 11, vorzugsweise 7 bis 9 und besonders bevorzugt 7 Kohlenstoffatomen ist, wobei die eine oder die mehreren Verbindungen der Formel (I) in einer Zusammensetzung enthaltend eine oder mehrere andere Substanzen ausgewählt aus der Gruppe bestehend aus Sorbitol, Sorbitolestern, Sorbitan, Sorbitanestern, Isosorbid, Isosorbiddiestern und Carbonsauren verwendet werden und die Zusammensetzung

I) 0,05 bis 0,7 Gewichtsteile Isosorbid und

II) 0,1 bis 1,0 Gewichtsteile an dem einem oder den mehreren Isosorbiddiestern der Formel (II),

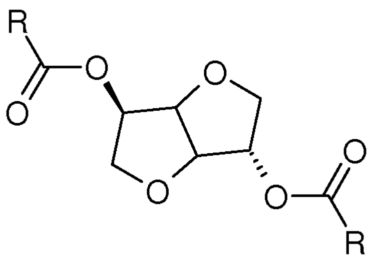

(II)

enthält, jeweils bezogen auf 1,0 Gewichtsteile an der einen oder den mehreren Verbindungen der Formel (I), wobei die OH-Zahl der Mischung aus der einen oder den mehreren Verbindungen der Formel (I) und der einen oder den mehreren Verbindungen ausgewählt aus der Gruppe bestehend aus Sorbitol, Sorbitolestern, Sorbitan, Sorbitanestern, Isosorbid, Isosorbiddiestern und Carbonsäuren kleiner oder gleich 320 ist.

Gegenstand der Erfindung ist somit die Verwendung einer oder mehrerer Verbindungen der Formel (I) in kosmetischen, dermatologischen oder pharmazeutischen Zusammensetzungen, in Pflanzenschutzformulierungen, in Wasch- oder Reinigungsmitteln oder in Farb-oder Anstrichmitteln,

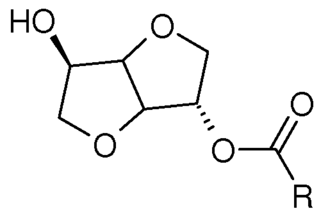

(I)

worin
R eine lineare oder verzweigte, gesättigte Alkylgruppe mit 5 bis 11, vorzugsweise 7 bis 9 und besonders bevorzugt 7 Kohlenstoffatomen oder eine lineare oder verzweigte, ein- oder mehrfach ungesättigte Alkenylgruppe mit 5 bis 11, vorzugsweise 7 bis 9 und besonders bevorzugt 7 Kohlenstoffatomen ist, als Verdicker, wobei die eine oder die mehreren Verbindungen der Formel (I) in einer Zusammensetzung enthaltend eine oder mehrere andere Substanzen ausgewählt aus der Gruppe bestehend aus Sorbitol, Sorbitolestern, Sorbitan, Sorbitanestern, Isosorbid, Isosorbiddiestern und Carbonsauren verwendet werden und die Zusammensetzung

I) 0,05 bis 0,7 Gewichtsteile Isosorbid und
II) 0,1 bis 1,0 Gewichtsteile an dem einem oder den mehreren Isosorbiddiestern der Formel (II),

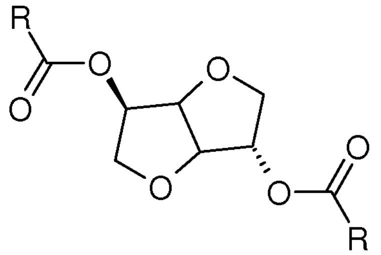

(II)

enthält, jeweils bezogen auf 1,0 Gewichtsteile an der einen oder den mehreren Verbindungen der Formel (I), wobei die OH-Zahl der Mischung aus der einen oder den mehreren Verbindungen der Formel (I) und der einen oder den mehreren Verbindungen ausgewählt aus der Gruppe bestehend aus Sorbitol, Sorbitolestern, Sorbitan, Sorbitanestern, Isosorbid, Isosorbiddiestern und Carbonsäuren kleiner oder gleich 320 ist.

Die Verbindungen der Formel (I) basieren auf nachwachsenden Rohstoffen wie z. B. auf Isosorbid, welches aus Zucker gewonnen werden kann. Ihre Herstellung ist in einfacher Weise nach dem Fachmann geläufigen Methoden, z. B. durch Veresterung von Isosorbid mit einer Säurekomponente wie einer Carbonsäure, möglich. Das Erscheinungsbild der Produkte, die die Verbindungen der Formel (I) enthalten, wird durch deren Anwesenheit nicht nachteilig beeinflusst. Beispielsweise können im Vergleich zur Verwendung von Xanthan Gum, welches als natürlicher Verdicker oftmals zu Produkten mit einer "brüchigen" bzw. unregelmäßigen oder nicht glatten Oberfläche führt, mit Hilfe der Verbindungen der Formel (I) verdickte Produkte hergestellt werden, die eine glatte Oberfläche aufweisen.
Zusammensetzungen, die zumindest zum Teil auf nachwachsenden Rohstoffen basieren und als Verdicker verwendet werden können, sind bereits bekannt.
WO 2010/108738 A2 (Evonik) beschreibt Formulierungen zur Reinigung und Pflege von menschlichen oder tierischen Körperteilen enthaltend Sorbitancarbonsäureester, wobei sich der Carbonsäureanteil des Sorbitancarbonsäureesters ableitet von einer Carbonsäure enthaltend 6 bis 10 Kohlenstoffatome und die Sorbitancarbonsäureester eine Hydroxylzahl (OH-Zahl) von größer 350 aufweisen sowie die Verwendung der genannten Sorbitancarbonsäureester als Viskositätsregler, Pflegewirkstoff, Schaumbooster oder Solubilisator in reinigenden oder pflegenden Formulierungen.
Weuthen et al in EP2239315A1 (Cognis IP Management GmbH) beschreibt Isosorbidmonoester und deren Verwendung im Haushalt und Paragraph 0005 offenbart Mischungen aus Mono- und Diestern.
Verbindungen der Formel (I) können z. B. nach dem Fachmann geläufigen Methoden hergestellt werden. Beispielsweise können die Verbindungen der Formel (I) durch Veresterung von Isosorbid nach üblichen und dem Fachmann bekannten Methoden hergestellt werden, wobei sowohl Isosorbid selbst als auch die zur Veresterung verwendeten Säurekomponenten wiederum käuflich erwerbbar sind.
Vorzugsweise ist der Rest R in der einen oder den mehreren Verbindungen der Formel (I) ein linearer gesättigter Alkylrest mit 7 bis 9 Kohlenstoffatomen.
Besonders bevorzugt ist der Rest R in der einen oder den mehreren Verbindungen der Formel (I) ein linearer gesättigter Alkylrest mit 7 Kohlenstoffatomen.

Die eine oder die mehreren Verbindungen der Formel (I) werden in Zusammensetzungen enthaltend eine oder mehrere andere Substanzen erfindungsgemäß als Verdicker verwendet. Diese Zusammensetzungen werden im Folgenden als "Zusammensetzungen A" bezeichnet.

Die Zusammensetzungen A enthalten eine oder mehrere Verbindungen der Formel (I) und zusätzlich eine oder mehrere andere Substanzen ausgewählt aus der Gruppe bestehend aus Sorbitol, Sorbitolestern (bei Sorbitolestern kann es sich um Mono-, Di-, Tri-, Tetra-, Penta- und/oder Hexaester handeln), Sorbitan, Sorbitanestern (bei Sorbitanestern kann es sich um Mono-, Di-, Tri- und/oder Tetraester handeln), Isosorbid, Isosorbiddiestern und Carbonsäuren. Bei "Sorbitan" kann es sich beispielsweise um 1,4- oder 1,5-Sorbitan handeln. Sowohl die Carbonsäuren selbst als auch die den Säurekomponenten der genannten Ester zu Grunde liegenden Carbonsäuren entsprechen der Formel RCOOH, worin R die bei Formel (I) angegebene Bedeutung besitzt und vorzugsweise ein linearer gesättigter Alkylrest mit 7 Kohlenstoffatomen ist, d. h. die Carbonsäure RCOOH vorzugsweise Caprylsäure ist.

Die Zusammensetzungen A enthalten eine oder mehrere Verbindungen der Formel (I) und zusätzlich

I) 0,05 bis 0,7 Gewichtsteile Isosorbid und
II) 0,1 bis 1,0 Gewichtsteile einen oder mehrere Isosorbiddiester der Formel (II)

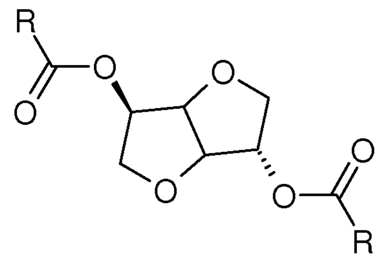

(II)

jeweils bezogen auf 1,0 Gewichtsteile an der einen oder den mehreren Verbindungen der Formel (I) und vorzugsweise bezogen auf 1,0 Gewichtsteile an Isosorbidmonocaprylat;
worin R die oben bei Formel (I) angegebene Bedeutung besitzt, und wobei der Isosorbiddiester vorzugsweise Isosorbiddicaprylat ist.

Hierunter wiederum bevorzugt enthalten die soeben genannten Zusammensetzungen A eine oder mehrere Verbindungen der Formel (I) und zusätzlich

I) 0,1 bis 0,7 bevorzugt 0,2 bis 0,5 Gewichtsteile Isosorbid und
II) 0,2 bis 1,0 bevorzugt 0,4 bis 0,8 Gewichtsteile an dem einen oder den mehreren Isosorbiddiestern der Formel (II), wobei der Isosorbiddiester vorzugsweise Isosorbiddicaprylat ist,

jeweils bezogen auf 1,0 Gewichtsteile an der einen oder den mehreren Verbindungen der Formel (I) und vorzugsweise bezogen auf 1,0 Gewichtsteile an Isosorbidmonocaprylat.

In einer hierunter wiederum bevorzugten Ausführungsform der Erfindung enthalten die soeben genannten Zusammensetzungen A entweder keine Carbonsäure RCOOH oder bis zu 0,1, vorzugsweise 0,001 bis 0,05 und besonders bevorzugt 0,002 bis 0,01 Gewichtsteile Carbonsäure RCOOH, wobei R die oben bei Formel (I) angegebene Bedeutung besitzt, und wobei die Carbonsäure vorzugsweise Caprylsäure ist, bezogen auf 1,0 Gewichtsteile an der einen oder den mehreren Verbindungen der Formel (I) und vorzugsweise bezogen auf 1,0 Gewichtsteile an Isosorbidmonocaprylat.

In einer weiteren besonders bevorzugten Ausführungsform der Erfindung enthalten die Zusammensetzungen A eine oder mehrere Verbindungen der Formel (I) und einen oder mehrere Sorbitanester aus Sorbitan und Carbonsäuren $R^aCOOH$, vorzugsweise ausgewählt aus Sorbitanestern aus 1,4- und/oder 1,5-Sorbitan und Carbonsäuren $R^aCOOH$, wobei $R^a$ eine lineare oder verzweigte, gesättigte Alkylgruppe mit 5 bis 11, vorzugsweise 7 bis 9 und besonders bevorzugt 7 Kohlenstoffatomen oder eine lineare oder verzweigte, ein- oder mehrfach ungesättigte Alkenylgruppe mit 5 bis 11, vorzugsweise 7 bis 9 und besonders bevorzugt 7 Kohlenstoffatomen ist, und wobei das Gewichtsverhältnis der einen oder der mehreren Verbindungen der Formel (I) zu dem einen oder den mehreren soeben genannten Sorbitanestern von 70 : 30 bis 100 : 0, vorzugsweise von 80 : 20 bis 100 : 0, besonders bevorzugt von 90 : 10 bis 100 : 0 und insbesondere bevorzugt von 95 : 5 bis 100 : 0 ist. Das angegebene Gewichtsverhältnis von "100 : 0" bedeutet, dass die soeben genannten Zusammensetzungen A in dieser besonders bevorzugten Ausführungsform der Erfindung keinen Sorbitanester enthalten müssen.

Unter den soeben genannten Zusammensetzungen A sind solche bevorzugt, worin der eine oder die mehreren

Sorbitanester aus Sorbitan und Carbonsäuren $R^aCOOH$ ausgewählt sind aus Sorbitanestern aus Sorbitan und Caprylsäure und vorzugsweise ausgewählt sind aus Sorbitanestern aus 1,4- und/oder 1,5-Sorbitan und Caprylsäure und der Sorbitanester besonders bevorzugt Sorbitanmonocaprylat ist.

In diesen Zusammensetzungen A ist die OH-Zahl der Mischung aus der einen oder den mehreren Verbindungen der Formel (I) und der einen oder den mehreren (gegebenenfalls enthaltenen) Sorbitanestern aus Sorbitan und Carbonsäuren $R^aCOOH$ vorzugsweise kleiner oder gleich 320, besonders bevorzugt kleiner oder gleich 285, insbesondere bevorzugt kleiner oder gleich 245 und außerordentlich bevorzugt kleiner oder gleich 225.

Die OH-Zahl der Mischung aus der einen oder den mehreren Verbindungen der Formel (I) und der einen oder den mehreren Verbindungen ausgewählt aus der Gruppe bestehend aus Sorbitol, Sorbitolestern, Sorbitan, Sorbitanestern, Isosorbid, Isosorbiddiestern und Carbonsäuren in den Zusammensetzungen A ist kleiner oder gleich 320, vorzugsweise kleiner oder gleich 285, besonders bevorzugt kleiner oder gleich 245 und insbesondere bevorzugt kleiner oder gleich 225.

In einer weiteren besonders bevorzugten Ausführungsform der Erfindung enthalten die Zusammensetzungen A keine Verbindungen ausgewählt aus Sorbitol und Sorbitolestern.

In einer weiteren besonders bevorzugten Ausführungsform der Erfindung enthalten die Zusammensetzungen A keine Verbindungen ausgewählt aus Sorbitan und Sorbitanestern.

Sofern die Zusammensetzungen A eine oder mehrere Verbindungen ausgewählt aus Sorbitol und Sorbitolestern (wobei die der Säurekomponente dieser Ester zu Grunde liegende Carbonsäure vorzugsweise Caprylsäure ist) enthalten, sind diese Verbindungen gemeinsam vorzugsweise in einer Menge kleiner oder gleich 5,0 Gew.-%, besonders bevorzugt in einer Menge kleiner oder gleich 3,0 Gew.-%, insbesondere bevorzugt in einer Menge kleiner oder gleich 1,0 Gew.-% und außerordentlich bevorzugt in einer Menge kleiner oder gleich 0,5 Gew.-% in den Zusammensetzungen A enthalten, wobei die Angaben in Gew.-% jeweils auf das Gesamtgewicht der fertigen Zusammensetzungen A bezogen sind.

Sofern die Zusammensetzungen A eine oder mehrere Verbindungen ausgewählt aus Sorbitan und Sorbitanestern (wobei die der Säurekomponente dieser Ester zu Grunde liegende Carbonsäure vorzugsweise Caprylsäure ist) enthalten, sind diese Verbindungen gemeinsam vorzugsweise in einer Menge kleiner oder gleich 20,0 Gew.-%, besonders bevorzugt in einer Menge kleiner oder gleich 10,0 Gew.-%, insbesondere bevorzugt in einer Menge kleiner oder gleich 5,0 Gew.-% und außerordentlich bevorzugt in einer Menge kleiner oder gleich 1,0 Gew.-% in den Zusammensetzungen A enthalten, wobei die Angaben in Gew.-% jeweils auf das Gesamtgewicht der fertigen Zusammensetzungen A bezogen sind.

In einer weiteren besonders bevorzugten Ausführungsform der Erfindung enthalten die Zusammensetzungen A die eine oder die mehreren Verbindungen der Formel (I) in Mengen von mindestens 30 Gew.-%, vorzugsweise in Mengen von mindestens 50 Gew.-% und besonders bevorzugt in Mengen von mindestens 60 Gew.-%, jeweils bezogen auf das Gesamtgewicht der fertigen Zusammensetzungen A.

Unter der Hydroxyl- oder OH-Zahl einer Substanz wird diejenige Menge KOH in mg verstanden, die der bei der Acetylierung von 1 g Substanz gebundenen Menge an Essigsäure äquivalent ist.

Geeignete Bestimmungsmethoden zur Ermittlung der OH-Zahl sind z. B. DGF C-V 17 a (53), Ph. Eur. 2.5.3 Method A und DIN 53240.

Im Rahmen der vorliegenden Erfindung werden die OH-Zahlen in Anlehnung an DIN 53240-2 bestimmt. Hierbei wird wie folgt vorgegangen: Es wird 1 g auf 0,1 mg genau von der homogenisierten zu messenden Probe eingewogen. 20,00 ml Acetylierungsgemisch (Acetylierungsgemisch: in 1 Liter Pyridin werden 50 ml Essigsäureanhydrid eingerührt) werden zugeben. Die Probe wird vollständig im Acetylierungsgemisch gelöst, gegebenenfalls unter Rühren und Erwärmen. 5 ml Katalysatorlösung (Katalysatorlösung: 2 g 4-Dimethylaminopyridin werden in 100 ml Pyridin gelöst) werden zugeben. Das Reaktionsgefäß wird verschlossen und 10 Minuten in das auf 55 °C vorgeheizte Wasserbad gestellt und dabei durchmischt. Die Reaktionslösung wird danach mit 10 ml vollentsalztem Wasser versetzt, das Reaktionsgefäß erneut verschlossen und nochmals im Schüttelwasserbad 10 Minuten abreagieren gelassen. Die Probe wird auf Raumtemperatur (25 °C) abgekühlt. Anschließend werden 50 ml 2-Propanol und 2 Tropfen Phenolphthalein zugeben. Diese Lösung wird mit Natronlauge (Natronlauge c = 0,5 mol/l) titriert (Va). Unter den gleichen Bedingungen, jedoch ohne Probeneinwaage, wird der Wirkwert des Acetylierungsgemisches bestimmt (Vb).

Aus dem Verbrauch der Wirkwertbestimmung und der Titration der Probe wird die OH-Zahl (OHZ) nach folgender Formel berechnet:

$$OHZ = \frac{(Vb - Va) \cdot c \cdot t \cdot M}{E}$$

OHZ = Hydroxylzahl in mg KOH/g Substanz
Va = Verbrauch an Natronlauge in ml bei der Titration der Probe

Vb = Verbrauch an Natronlauge in ml bei der Titration des Wirkwertes
c = Stoffmengenkonzentration der Natronlauge in mol/l
t = Titer der Natronlauge
M = Molare Masse von KOH = 56,11 g/mol
E = Probeneinwaage in g

(Vb - Va) ist diejenige Menge der verwendeten Natronlauge in ml, die der bei der oben beschriebenen Acetylierung der zu messenden Probe gebundenen Menge an Essigsäure äquivalent ist.

Die soeben beschriebene Methode zur Bestimmung der OH-Zahl wird im Folgenden als "Methode OHZ-A" bezeichnet.

Die erfindungsgemäße Verwendung findet in kosmetischen, dermatologischen oder pharmazeutischen Zusammensetzungen, in Pflanzenschutzformulierungen, in Wasch- oder Reinigungsmitteln oder in Farb- oder Anstrichmitteln statt. Die Pflanzenschutzmittel enthalten ein oder mehrere Pestizide.

Die kosmetischen, dermatologischen oder pharmazeutischen Zusammensetzungen, die Pflanzenschutzformulierungen, die Wasch- oder Reinigungsmittel oder die Farb- oder Anstrichmittel enthalten die eine oder die mehreren Verbindungen der Formel (I) in Mengen von 0,01 bis 10,0 Gew.-%, bevorzugt in Mengen von 0,1 bis 5,0 Gew.-% und insbesondere bevorzugt in Mengen von 0,2 bis 3,0 Gew.-%, jeweils bezogen auf das Gesamtgewicht der fertigen kosmetischen, dermatologischen oder pharmazeutischen Zusammensetzungen, Pflanzenschutzformulierungen, Wasch- oder Reinigungsmittel oder Farb- oder Anstrichmittel.

Die kosmetischen, dermatologischen oder pharmazeutischen Zusammensetzungen, die Pflanzenschutzformulierungen, die Wasch- oder Reinigungsmittel oder die Farb- oder Anstrichmittel haben Viskositäten vorzugsweise im Bereich von 50 bis 200 000 mPa · s, besonders bevorzugt im Bereich von 500 bis 100 000 mPa · s, insbesondere bevorzugt im Bereich von 2 000 bis 50 000 mPa · s und außerordentlich bevorzugt im Bereich von 5 000 bis 30 000 mPa · s (20 °C, Brookfield RVT, RV-Spindel-Satz; 20 Umdrehungen pro Minute).

Die kosmetischen, dermatologischen oder pharmazeutischen Zusammensetzungen liegen vorzugsweise in Form von Fluids, Gelen, Schäumen, Sprays, Lotions oder Cremes vor.

Die kosmetischen, dermatologischen oder pharmazeutischen Zusammensetzungen, die Pflanzenschutzformulierungen, die Wasch- oder Reinigungsmittel oder die Farb- oder Anstrichmittel sind vorzugsweise auf wässriger oder wässrig-alkoholischer Basis aufgebaut oder liegen als Emulsionen oder Dispersionen vor. Besonders bevorzugt liegen sie als Emulsionen vor und insbesondere bevorzugt liegen sie als Öl-in-Wasser Emulsionen vor.

Die kosmetischen, dermatologischen oder pharmazeutischen Zusammensetzungen, die Pflanzenschutzformulierungen, die Wasch- oder Reinigungsmittel oder die Farb- oder Anstrichmittel können als weitere Hilfs- und Zusatzstoffe alle für die jeweilige Anwendung üblicherweise verwendeten Substanzen enthalten, beispielsweise Öle, Wachse, Emulgatoren, Co-Emulgatoren, Dispergatoren, Tenside, Entschäumer, Solubilisatoren, Elektrolyte, Hydroxysäuren, Stabilisatoren, Polymere, Filmbildner, weitere (von den Verbindungen der Formel (I) verschiedene) Verdicker, Gelierungsmittel, Überfettungsmittel, Rückfetter, antimikrobielle Wirkstoffe, biogene Wirkstoffe, Adstringentien, Aktivstoffe, deodorierende Stoffe, Sonnenschutzfilter, Antioxidantien, Oxidantien, Feuchthaltemittel, Lösungsmittel, Farbmittel, Pigmente, Perlglanzmittel, Duftstoffe, Trübungsmittel und/oder Silikone.

Die kosmetischen, dermatologischen oder pharmazeutischen Zusammensetzungen, die Pflanzenschutzformulierungen, die Wasch- oder Reinigungsmittel oder die Farb- oder Anstrichmittel besitzen pH-Werte von vorzugsweise 2 bis 11, besonders bevorzugt von 4,5 bis 8,5 und insbesondere bevorzugt von 5,5 bis 6,5.

Die nachfolgenden Beispiele und Anwendungen sollen die Erfindung näher erläutern, ohne sie jedoch darauf zu beschränken. Bei allen Prozentangaben handelt es sich um Gewichts-% (Gew.-%), sofern nicht explizit anders angegeben.

Verdickungsleistungen wurden unter folgenden Bedingungen gemessen: Brookfield RVT; 20 °C; RV-Spindel-Satz; 20 Umdrehungen pro Minute

Versuchsbeispiele:

A) Herstellung von Isosorbidcaprylat

In einer Rührapparatur mit Destillationsaufsatz werden 190,0 g (1,3 mol) Isosorbid ("Sorbon" von Ecogreen Oleochemicals) und 187,5 g (1,3 mol) Octansäure (Caprylsäure) bei 80 °C zusammen mit 0,38 g Natronlauge (18 Gew.-%ig, wässrig) als Katalysator vorgelegt. Unter Rühren und Stickstoffüberleitung (10 - 12 Liter pro Stunde) wird das Reaktionsgemisch zunächst auf 180 °C aufgeheizt, wobei das Reaktionswasser abzudestillieren beginnt. Der Ansatz wird dann in 1 Stunde auf 190 °C und in weiteren 2 Stunden auf 210 °C aufgeheizt. Nach Erreichen von 210 °C wird solange verestert bis eine Säurezahl von < 1 mg KOH/g erreicht ist. Man erhält 345,7 g bernsteinfarbenes Isosorbidcaprylat (97 % der Theorie). Der pH-Wert (5 Gew.-% in Ethanol/Wasser 1:1) beträgt 5,9. Der pH-Wert wurde gemessen

gemäß DIN EN 1262.

Weitere analytische Kenndaten des Isosorbidcaprylats:

| | |
|---|---|
| Säurezahl: | 0,9 mg KOH/g, gemessen gemäß DIN EN ISO 2114 |
| Hydroxyl-Zahl: | 206 mg KOH/g, gemessen in Anlehnung an DIN 53240-2 nach Methode OHZ-A |
| Verseifungszahl: | 204 mg KOH/g, gemessen gemäß DIN EN ISO 3681 |

Das Isosorbidcaprylat besitzt folgende Zusammensetzung:

| Substanz | Gew.-% |
|---|---|
| Caprylsäure | 0,4 |
| Isosorbid | 18,1 |
| Isosorbidmonocaprylat | 50,9 |
| Isosorbiddicaprylat | 30,6 |

Diese Zusammensetzung wird im Folgenden als "Isosorbidcaprylat 1" bezeichnet.

B) Bestimmung der Verdickungsleistung von Isosorbidcaprylat 1

Unter Verwendung von Genapol® LRO (Sodium Laureth-2 Sulfate, 27 Gew.-% in Wasser) und Genagen® CAB 818 (Cocamidopropylbetain, 30 Gew.-% in Wasser) und zusätzlich Wasser wurde eine Mischung hergestellt, die die beiden Tenside im Gewichtsverhältnis 8 : 2 zu 15 Gew.-% in Wasser enthält (im Folgenden "Mischung 1" genannt) . Zu dieser Mischung 1 wurden verschiedene Substanzen zugegeben und die Visosität der resultierenden Zusammensetzungen bestimmt. Die Ergebnisse sind in der folgenden Tabelle 1 wiedergegeben.

Tabelle 1 gemessene Viskositäten

| Zugegebene Substanz; Menge [Gew.-%] | Viskosität [mPa · s] |
|---|---|
| NaCl [0,5 Gew.-%] | 40 |
| NaCl [0,5 Gew.-%] und Isosorbidcaprylat 1 [1 Gew.-%] | 330 |

Zu Mischung 1 wurde Isosorbidcaprylat 1 in unterschiedlichen Mengen zugegeben und die Viskosität der resultierenden Zusammensetzungen bestimmt. Die Ergebnisse sind in der folgenden Tabelle 2 wiedergegeben.

Tabelle 2 gemessene Viskositäten

| Menge an zugegebenem Isosorbidcaprylat 1 [Gew.-%] | Viskosität [mPa · s] |
|---|---|
| 0 | 5 |
| 0,2 | 6 |
| 0,4 | 10 |
| 0,6 | 12 |
| 0,8 | 21 |
| 1,0 | 12 |
| 1,2 | 37 |
| 1,4 | 34 |
| 1,6 | 45 |
| 1,8 | 55 |
| 2,0 | 72 |

An den in Tabellen 1 und 2 aufgeführten Ergebnissen erkennt man, dass Isosorbidcaprylat 1 eine verdickende Wirkung in wässrigen tensidischen Systemen besitzt.

C) Bestimmung der Verdickungsleistung von Isosorbidcaprylat 1

Unter Verwendung von Genapol® LRO (Sodium Laureth-2 Sulfate, 27 Gew.-% in Wasser) und Genagen® KB (Cocobetain, 30 Gew.-% in Wasser) und zusätzlich Wasser wurde eine Mischung hergestellt, die die beiden Tenside im Gewichtsverhältnis 8 : 2 zu 15 Gew.-% in Wasser enthält (im Folgenden "Mischung 2" genannt) . Zu dieser Mischung 2 wurden verschiedene Substanzen zugegeben und die Visosität der resultierenden Zusammensetzungen bestimmt. Die Ergebnisse sind in der folgenden Tabelle 3 wiedergegeben.

Tabelle 3 gemessene Viskositäten

| Zugegebene Substanz; Menge [Gew.-%] | Viskosität [mPa · s] |
|---|---|
| NaCl [0,5 Gew.-%] | 3830 |
| NaCl [0,5 Gew.-%] und Isosorbidcaprylat 1 [1 Gew.-%] | 14200 |

Zu Mischung 2 wurde Isosorbidcaprylat 1 in unterschiedlichen Mengen zugegeben und die Viskosität der resultierenden Zusammensetzungen bestimmt. Die Ergebnisse sind in der folgenden Tabelle 4 wiedergegeben.

Tabelle 4 gemessene Viskositäten

| Menge an zugegebenem Isosorbidcaprylat 1 [Gew.-%] | Viskosität [mPa · s] |
|---|---|
| 0 | 135 |
| 0,2 | 310 |
| 0,4 | 460 |
| 0,6 | 875 |
| 0,8 | 1550 |
| 1,0 | 2510 |
| 1,2 | 4190 |
| 1,4 | 5650 |
| 1,6 | 6600 |
| 1,8 | 7400 |
| 2,0 | 7050 |

An den in Tabellen 3 und 4 aufgeführten Ergebnissen erkennt man, dass Isosorbidcaprylat 1 eine verdickende Wirkung in wässrigen tensidischen Systemen besitzt.

D) Bestimmung der Verdickungsleistung von Isosorbidcaprylat 1

Es wurde eine Emulsion wie in der folgenden Tabelle 5 angegeben hergestellt und Isosorbidcaprylat 1 in unterschiedlichen Konzentrationen zugegeben. Es wurden die Viskositäten der resultierenden Emulsionen bestimmt. Die Ergebnisse sind in der folgenden Tabelle 5 wiedergegeben.

Tabelle 5 gemessene Viskositäten

| Zusammensetzung | 1 | 2 | 3 | 4 |
|---|---|---|---|---|
| Hostaphat® KL 340 D (A) Trilaureth-4 Phosphate | 1 | 1 | 1 | 1 |
| Mineral Oil n.v.(A) | 8 | 8 | 8 | 8 |
| Isopropyl Palmitate (A) | 3 | 3 | 3 | 3 |
| Lanette® O (A) Cetearyl Alcohol | 0,5 | 0,5 | 0,5 | 0,5 |

(fortgesetzt)

| | | | | |
|---|---|---|---|---|
| Myritol® 318 (A) Caprylic/Capric Triglyceride | 2 | 2 | 2 | 2 |
| Tegin® M (A) Glyceryl Stearate | 0,5 | 0,5 | 0,5 | 0,5 |
| Isosorbidcaprylat 1 (A) | - | 0,5 | 1,0 | 1,5 |
| Aristoflex® AVC (B) Ammonium Acryloyldimethyltaurate/VP Copolymer | 0,4 | 0,4 | 0,4 | 0,4 |
| Wasser (C) | 71,6 | 72,1 | 71,6 | 71,1 |
| Glycerin (C) | 5 | 5 | 5 | 5 |
| Alcohol (D) | 5 | 5 | 5 | 5 |
| Tocopheryl Acetate (D) | 1 | 1 | 1 | 1 |
| SilCare® Silicone 41 M15 (D) Caprylyl Methicone | 1 | 1 | 1 | 1 |
| Nipaguard® PDU (E) Diazolidinyl Urea, Methylparaben, Propylparaben, Propylenglykol | 1 | 1 | 1 | 1 |
| | | | | |
| pH gemessen | 6,34 | 6,21 | 6,12 | 6,20 |
| | | | | |
| Viskosität [mPa · s] | 1860 | 2260 | 3130 | 3200 |

Herstellung:

I Mische die Komponenten von A und erhitze auf 60 °C
II Mische die Komponenten von C und erhitze auf 60 °C
III Gebe B zu I
IV Gebe II zu III und Rühre bis die Mischung auf Raumtemperatur abgekühlt ist
V Gebe die Komponenten D nach und nach zu IV
VI Gebe E zu V und homogenisiere die Emulsion

An den in Tabelle 5 aufgeführten Ergebnissen erkennt man, dass Isosorbidcaprylat 1 eine verdickende Wirkung in Emulsionen besitzt.

E) Vergleichsversuch

Es wurde die Verdickungsleistung von Isosorbidcaprylat 1 in Mischung 2 mit einer Zusammensetzung enthaltend 89,6 Gew.-% Isosorbiddicaprylat und 9,4 Gew.-% Isosorbidmonocaprylat (Rest: 1 Gew.-%) (im Folgenden "Isosorbiddicaprylat" genannt) einerseits und mit reinem Isosorbid andererseits verglichen. Die Ergebnisse sind in Tabelle 6 dargestellt.

Tabelle 6 gemessene Viskositäten

| Zu Mischung 2 zugegebene Substanz; Menge [Gew.-%] | Viskosität [mPa · s] |
|---|---|
| Keine | 135 |
| Isosorbidcaprylat 1 [1 Gew.-%] | 2510 |
| Isosorbiddicaprylat [1 Gew.-%] | 2390 |
| Isosorbid [1 Gew.-%] | 160 |

Wie den Ergebnissen der Tabelle 6 zu entnehmen ist, besitzt Isosorbid keine nennenswerte Verdickungsleistung, während Isosorbiddicaprylat eine signifikante Verdickung bewirkt.

Wie den Ergebnissen der Tabelle 6 aber ebenfalls zu entnehmen ist, bewirkt Isosorbidcaprylat 1 eine vergleichbare Verdickung wie Isosorbiddicaprylat. Würde die Verdickungsleistung von Isosorbidcaprylat 1 lediglich auf das darin

enthaltene Isosorbiddicaprylat zurückzuführen sein, müsste die Viskosität bei Verwendung von Isosorbidcaprylat 1 deutlich geringer sein als bei Verwendung von Isosorbiddicaprylat, weil Isosorbidcaprylat 1 lediglich 30,6 Gew.-% an Isosorbiddicaprylat enthält. Da das in Isosorbidcaprylat 1 enthaltene Isosorbid (wie bereits oben erwähnt) keine nennenswerte Verdickungsleistung zeigt und die eventuelle Verdickung durch die in Isorbidcaprylat 1 enthaltene Caprylsäure auf Grund ihrer geringen Menge vernachlässigbar ist, aber die Verdickungsleistung von Isosorbidcaprylat 1 im Vergleich zur Verdickungsleistung von Isosorbiddicaprylat trotzdem vergleichbar ist, muss die in Isosorbidcaprylat 1 enthaltene Verbindung Isorbidmonocaprylat eine signifikante Verdickungsleistung besitzen.

F) Anwendungsbeispiele

Die erfindungsgemäße Verwendung kann beispielsweise in folgenden Formulierungen stattfinden.

Formulierungbeispiel 1: Sehr mildes EO- und Sulfat-freies Shampoo

| Phase | Inhaltsstoff | Gew.-% |
|---|---|---|
| A | Wasser | ad 100 |
| B | Sorbitol | 1,0 |
| C | Hostapon® SG<br>Sodium Cocoyl Glycinate<br>Genagen® KB<br>Coco-Betaine<br>Plantacare® 818 UP<br>Coco Glucoside<br>Isosorbidcaprylat 1 | 30,0<br><br>15,0<br><br>9,23<br><br>1,0 |
| D | Lactic Acid 25 Gew.-%ig in Wasser | 3,25 |
| E | Konservierungsmittel | q.s. |

Herstellung:

I Gebe B zu A und rühre bis eine klare Lösung erhalten wird
II Gebe C zu I und rühre bis die Lösung homogen ist
III Stelle den pH-Wert mit D auf 7,0-7,2 ein
IV Gebe E zu III

Formulierungsbeispiel 2: Milde Gesichtsreinigung

| Phase | Inhaltsstoff | Gew.-% |
|---|---|---|
| A | Wasser<br>Glycerin | ad 100<br>20,0 |
| B | Hostapon® SG<br>Sodium Cocoyl Glycinate<br>Lauric Acid<br>Myristic Acid<br>Stearic Acid<br>Isosorbidcaprylat 1 | 34,0<br><br>1,0<br>0,25<br>0,25<br>1,0 |
| C | Lactic Acid<br>Wasser | 0,90<br>6,80 |
| D | Genagen® CAB<br>Cocamidopropyl Betaine | 10,0 |

(fortgesetzt)

| Phase | Inhaltsstoff | Gew.-% |
|---|---|---|
| E | Konservierungsmittel<br>Duftstoff | q.s.<br>q.s. |

Herstellung:

I Mische Phase A und erwärme auf 80 °C
II Gebe Komponenten von B nach und nach zu I und rühre weiter bei 80 °C
III Mische Phase C und gebe zu II
IV Lasse auf 60 °C abkühlen und gebe D zu III
V Bei 40 °C gebe E zu IV

Formulierungsbeispiel 3: Handgeschirrspülmittel

| Inhaltsstoff | Gew.-% |
|---|---|
| Hostapur® SAS 60 (Alkansulfonat, 60 Gew.-% in Wasser) | 40,0 |
| Hostapur® OS liquid (Sodium C14-16 Alkyl Sulfonate, 40 Gew.-% in Wasser) | 11,0 |
| Isosorbidcaprylat 1 | 1,0 |
| Genagen® CAB (Cocoamidopropylbetain, 30 Gew.-% in Wasser) | 3,0 |
| Konservierungsmittel | q.s. |
| Wasser | ad 100 |

Formulierungsbeispiel 4: Oberflächenreiniger (Allzweckreiniger)

| Inhaltsstoff | Gew.-% |
|---|---|
| Hostapur® SAS 60 (Alkansulfonat, 60 Gew.-% in Wasser) | 5,0 |
| Genapol® UD 080 (Undecanol + 8 EO) | 2,0 |
| Isosorbidcaprylat 1 | 1,0 |
| Konservierungsmittel | q.s. |
| Wasser | ad 100 |

Herstellung der Formulierungsbeispiele 3 und 4:

Die Hälfte der Wassermenge wird vorgelegt und die Komponenten werden in der Reihenfolge wie in den Tabellen aufgeführt eingerührt. Die restliche Menge Wasser wird dann nachgegeben. Es resultieren klare, wässrige Zusammensetzungen.

## Patentansprüche

1. Verwendung einer oder mehrerer Verbindungen der Formel (I) in kosmetischen, dermatologischen oder pharmazeutischen Zusammensetzungen, in Pflanzenschutzformulierungen, in Wasch- oder Reinigungsmitteln oder in Farb- oder Anstrichmitteln,

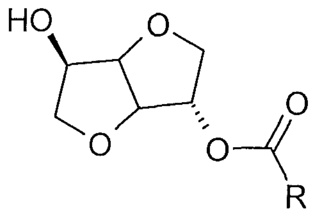

(I)

worin

R eine lineare oder verzweigte, gesättigte Alkylgruppe mit 5 bis 11 Kohlenstoffatomen oder eine lineare oder verzweigte, ein- oder mehrfach ungesättigte Alkenylgruppe mit 5 bis 11 Kohlenstoffatomen ist,

als Verdicker, wobei die eine oder die mehreren Verbindungen der Formel (I) in einer Zusammensetzung enthaltend eine oder mehrere andere Substanzen ausgewählt aus der Gruppe bestehend aus Sorbitol, Sorbitolestern, Sorbitan, Sorbitanestern, Isosorbid, Isosorbiddiestern und Carbonsäuren verwendet werden und die Zusammensetzung

I) 0,05 bis 0,7 Gewichtsteile Isosorbid und
II) 0,1 bis 1,0 Gewichtsteile an dem einem oder den mehreren Isosorbiddiestern der Formel (II),

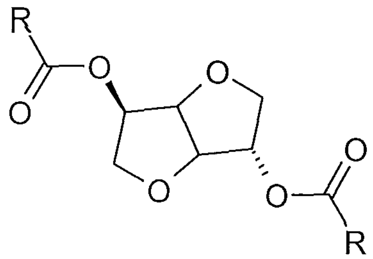

(II)

enthält, jeweils bezogen auf 1,0 Gewichtsteile an der einen oder den mehreren Verbindungen der Formel (I), wobei die OH-Zahl der Mischung aus der einen oder den mehreren Verbindungen der Formel (I) und der einen oder den mehreren Verbindungen ausgewählt aus der Gruppe bestehend aus Sorbitol, Sorbitolestern, Sorbitan, Sorbitanestern, Isosorbid, Isosorbiddiestern und Carbonsäuren kleiner oder gleich 320 ist.

2. Verwendung nach Anspruch 1, **dadurch gekennzeichnet, dass** der Rest R in Formel (I) ein linearer gesättigter Alkylrest mit 7 bis 9 Kohlenstoffatomen ist.

3. Verwendung nach Anspruch 2, **dadurch gekennzeichnet, dass** der Rest R in Formel (I) ein linearer gesättigter Alkylrest mit 7 Kohlenstoffatomen ist.

4. Verwendung nach einem oder mehreren der Ansprüche 1 bis 3, **dadurch gekennzeichnet, dass** die Zusammensetzung eine oder mehrere Verbindungen der Formel (I) und einen oder mehrere Sorbitanester aus Sorbitan und Carbonsäuren $R^aCOOH$, wobei $R^a$ eine lineare oder verzweigte, gesättigte Alkylgruppe mit 5 bis 11 Kohlenstoffatomen oder eine lineare oder verzweigte, ein- oder mehrfach ungesättigte Alkenylgruppe mit 5 bis 11 Kohlenstoffatomen ist, enthält, und das Gewichtsverhältnis der einen oder der mehreren Verbindungen der Formel (I) zu dem einen oder den mehreren soeben genannten Sorbitanestern von 70 : 30 bis 100 : 0 ist.

5. Verwendung nach Anspruch 4, **dadurch gekennzeichnet, dass** der eine oder die mehreren Sorbitanester aus Sorbitan und Carbonsäuren $R^aCOOH$ ausgewählt sind aus Sorbitanestern aus Sorbitan und Caprylsäure.

6. Verwendung nach einem oder mehreren der Ansprüche 4 bis 5, **dadurch gekennzeichnet, dass** die Zusammensetzung die eine oder die mehreren Verbindungen der Formel (I) in Mengen von mindestens 30 Gew.-% enthält, jeweils bezogen auf das Gesamtgewicht der fertigen Zusammensetzung.

7. Verwendung nach einem oder mehreren der Ansprüche 1 bis 6, **dadurch gekennzeichnet, dass** die kosmetischen, dermatologischen oder pharmazeutischen Zusammensetzungen, die Pflanzenschutzformulierungen, die Wasch- oder Reinigungsmittel oder die Farb- oder Anstrichmittel die eine oder die mehreren Verbindungen der Formel (I)

in Mengen von 0,01 bis 10,0 Gew.-% enthalten, jeweils bezogen auf das Gesamtgewicht der fertigen kosmetischen, dermatologischen oder pharmazeutischen Zusammensetzungen, Pflanzenschutzformulierungen, Wasch- oder Reinigungsmittel oder Farb- oder Anstrichmittel.

8. Verwendung nach einem oder mehreren der Ansprüche 1 bis 7, **dadurch gekennzeichnet, dass** die kosmetischen, dermatologischen oder pharmazeutischen Zusammensetzungen, die Pflanzenschutzformulierungen, die Wasch- oder Reinigungsmittel oder die Farb- oder Anstrichmittel auf wässriger oder wässrig-alkoholischer Basis aufgebaut sind oder als Emulsion oder Dispersion vorliegen.

9. Verwendung nach einem oder mehreren der Ansprüche 1 bis 8, **dadurch gekennzeichnet, dass** die kosmetischen, dermatologischen oder pharmazeutischen Zusammensetzungen, die Pflanzenschutzformulierungen, die Wasch- oder Reinigungsmittel oder die Farb- oder Anstrichmittel einen pH-Wert von 2 bis 11 besitzen.

## Claims

1. Use of one or more compounds of the formula (I) in cosmetic, dermatological or pharmaceutical compositions, in crop protection formulations, in washing or cleaning compositions or in paints or coatings,

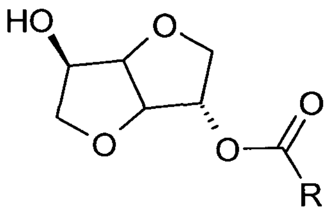

(I)

in which
R is a straight-chain or branched saturated alkyl group having 5 to 11 carbon atoms or a straight-chain or branched mono- or polyunsaturated alkenyl group having 5 to 11 carbon atoms as thickeners, wherein the one or more compounds of the formula (I) are used in a composition comprising one or more other substances selected from the group consisting of sorbitol, sorbitol esters, sorbitan, sorbitan esters, isosorbide, isosorbide diesters and carboxylic acids and the composition comprises

I) from 0.05 to 0.7 part by weight of isosorbide and
II) from 0.1 to 1.0 part by weight of the one or more isosorbide diesters of the formula (II),

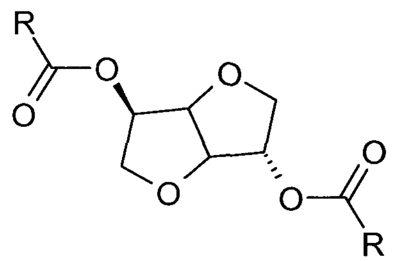

(II)

in each case based on 1.0 part by weight of the one or more compounds of the formula (I), where the hydroxyl value of the mixture of the one or more compounds of the formula (I) and the one or more compounds selected from the group consisting of sorbitol, sorbitol esters, sorbitan, sorbitan esters, isosorbide, isosorbide diesters and carboxylic acids is smaller than or equal to 320.

2. The use as claimed in claim 1, wherein the radical R in formula (I) is a straight-chain saturated alkyl radical having 7 to 9 carbon atoms.

3. The use as claimed in claim 2, wherein the radical R in formula (I) is a straight-chain saturated alkyl radical having 7 carbon atoms.

**4.** The use as claimed in one or more of claims 1 to 3, wherein the composition comprises one or more compounds of the formula (I) and one or more sorbitan esters of sorbitan and carboxylic acids $R^aCOOH$, where $R^a$ is a straight-chain or branched saturated alkyl group having 5 to 11 carbon atoms or a straight-chain or branched mono- or polyunsaturated alkenyl group having 5 to 11 carbon atoms, and the weight ratio of the one or more compounds of the formula (I) to the one or more sorbitan esters just mentioned is from 70 : 30 to 100 : 0.

**5.** The use as claimed in claim 4, wherein the one or more sorbitan esters of sorbitan and carboxylic acids $R^aCOOH$ are selected from sorbitan esters of sorbitan and caprylic acid.

**6.** The use as claimed in one or more of claims 4 to 5, wherein the composition comprises the one or more compounds of the formula (I) in amounts of at least 30% by weight, in each case based on the total weight of the finished composition.

**7.** The use as claimed in one or more of claims 1 to 6, wherein the cosmetic, dermatological or pharmaceutical compositions, the crop protection formulations, the washing or cleaning compositions or the paints or coatings comprise the one or more compounds of the formula (I) in amounts of from 0.01 to 10.0% by weight, in each case based on the total weight of the finished cosmetic, dermatological or pharmaceutical compositions, crop protection formulations, washing or cleaning compositions or paints or coatings.

**8.** The use as claimed in one or more of claims 1 to 7, wherein the cosmetic, dermatological or pharmaceutical compositions, the crop protection formulations, the washing or cleaning compositions or the paints or coatings are formulated on an aqueous or aqueous-alcoholic basis or are present as emulsion or dispersion.

**9.** The use as claimed in one or more of claims 1 to 8, wherein the cosmetic, dermatological or pharmaceutical compositions, the crop protection formulations, the washing or cleaning compositions or the paints or coatings have a pH of from 2 to 11.

**Revendications**

**1.** Utilisation d'un ou de plusieurs composés de formule (I) dans des compositions cosmétiques, dermatologiques ou pharmaceutiques, dans des formulations phytosanitaires, dans des détergents ou dans des colorants ou des peintures,

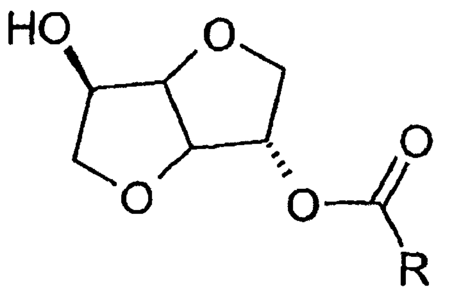

(I)

dans laquelle
R représente un groupe alkyle saturé, linéaire ou ramifié, de 5 à 11 atomes de carbone ou un groupe alcényle linéaire ou ramifié, mono- ou polyinsaturé, de 5 à 11 atomes de carbone,
en tant qu'épaississant, le ou les composés de formule (I) étant utilisés dans une composition contenant une ou plusieurs autres substances choisies dans le groupe constitué par le sorbitol, les esters de sorbitol, le sorbitane, les esters de sorbitane, l'isosorbide, les diesters d'isosorbide et les acides carboxyliques, et la composition contenant

I) 0,05 à 0,7 partie en poids d'isosorbide et
II) 0,1 à 1,0 partie en poids du ou des diesters d'isosorbide de formule (II)

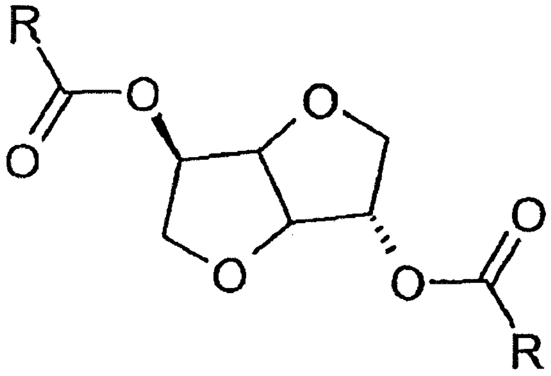

(II)

à chaque fois par rapport à 1,0 partie en poids du ou des composés de formule (I), l'indice OH du mélange du ou des composés de formule (I) et du ou des composés choisis dans le groupe constitué par le sorbitol, les esters de sorbitol, le sorbitane, les esters de sorbitane, l'isosorbide, les diesters d'isosorbide et les acides carboxyliques étant inférieur ou égal à 320.

2. Utilisation selon la revendication 1, **caractérisée en ce que** le radical R dans la formule (I) est un radical alkyle linéaire saturé de 7 à 9 atomes de carbone.

3. Utilisation selon la revendication 2, **caractérisée en ce que** le radical R dans la formule (I) est un radical alkyle linéaire saturé de 7 atomes de carbone.

4. Utilisation selon une ou plusieurs des revendications 1 à 3, **caractérisée en ce que** la composition contient un ou plusieurs composés de formule (I) et un ou plusieurs esters de sorbitane constitués de sorbitane et d'acides carboxyliques $R^aCOOH$, $R^a$ étant un groupe alkyle saturé linéaire ou ramifié de 5 à 11 atomes de carbone ou un groupe alcényle linéaire ou ramifié, mono- ou polyinsaturé, de 5 à 11 atomes de carbone, et le rapport en poids entre le ou les composés de formule (I) et ledit ou lesdits esters de sorbitane est de 70:30 à 100:0.

5. Utilisation selon la revendication 4, **caractérisée en ce que** le ou les esters de sorbitane constitués de sorbitane et d'acides carboxyliques $R^aCOOH$ sont choisis parmi les esters de sorbitane constitués de sorbitane et d'acide caprylique.

6. Utilisation selon une ou plusieurs des revendications 4 à 5, **caractérisée en ce que** la composition contient le ou les composés de formule (I) en quantités d'au moins 30 % en poids, à chaque fois par rapport au poids total de la composition finie.

7. Utilisation selon une ou plusieurs des revendications 1 à 6, **caractérisée en ce que** les compositions cosmétiques, dermatologiques ou pharmaceutiques, les formulations phytosanitaires, les détergents ou les colorants ou peintures contiennent le ou les composés de formule (I) en quantités de 0,01 à 10,0 % en poids, à chaque fois par rapport au poids total des compositions cosmétiques, dermatologiques ou pharmaceutiques, des formulations phytosanitaires, des détergents ou des colorants ou peintures finis.

8. Utilisation selon une ou plusieurs des revendications 1 à 7, **caractérisée en ce que** les compositions cosmétique, dermatologiques ou pharmaceutiques, les formulations phytosanitaires, les détergents ou les colorants ou peintures sont à base aqueuse ou aqueuse-alcoolique, ou se présentent sous la forme d'une émulsion ou d'une dispersion.

9. Utilisation selon une ou plusieurs des revendications 1 à 8, **caractérisée en ce que** les compositions cosmétiques, dermatologiques ou pharmaceutiques, les formulations phytosanitaires, les détergents ou les colorants ou peintures présentent un pH de 2 à 11.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010108738 A2
- EP 2239315 A1, Weuthen